**Europäisches Patentamt**

**European Patent Office**

**Office européen des brevets**

(19)

(11) Veröffentlichungsnummer: **0 130 530**
**B1**

(12)

# EUROPÄISCHE PATENTSCHRIFT

(45) Veröffentlichungstag der Patentschrift:
**24.06.87**

(21) Anmeldenummer: **84107310.9**

(22) Anmeldetag: **26.06.84**

(51) Int. Cl.⁴: **C 12 Q 1/04,** C 12 N 5/00,
C 12 N 13/00, G 01 N 33/00

(54) **Verfahren und Vorrichtung zur Bestimmung von Zellinhaltstoffe sezernierenden Zellen.**

(30) Priorität: **29.06.83 DE 3323415**

(43) Veröffentlichungstag der Anmeldung:
**09.01.85 Patentblatt 85/2**

(45) Bekanntmachung des Hinweises auf die Patenterteilung:
**24.06.87 Patentblatt 87/26**

(84) Benannte Vertragsstaaten:
**AT BE CH FR GB IT LI LU NL SE**

(56) Entgegenhaltungen:
**DD - B - 160 229**

**ZEITSCHRIFT FÜR NATURFORSCHUNG, Band 37c, Nr.
10, Oktober 1982, Tübingen. W. M. ARNOLD et al.:
"Rotating-Field-Induced Rotation and Measurement of
the Membrane Capacitance of Single Mesophyll Cells",
Seiten 908-915**

(73) Patentinhaber: **Kernforschungsanlage Jülich
Gesellschaft mit beschränkter Haftung, Postfach 1913,
D-5170 Jülich 1 (DE)**

(72) Erfinder: **Arnold, William Michael,
Adalbertstrasse 116-118, D-5100 Aachen (DE)**
Erfinder: **Zimmermann, Ulrich, Prof., Im Geyberg 21,
D-5165 Hürtgenwald-Gey (DE)**

## Beschreibung

Die Erfindung bezieht sich auf ein Verfahren zur Unterscheidung von Zellen, die Zellinhaltstoffe, wie Proteine und/oder Glycoproteine, Hormone oder sog. Wachstums-Faktoren sezernieren, von Zellen der gleichen Art oder Gattung, die keine Zellinhaltstoffe sezernieren.

In einer Vielzahl von Anwendungsfällen im biologischen, pharmakologischen und medizinischen Bereich besteht das Bedürfnis, Zellinhaltstoffe sezernierender Zellen von Zellen der gleichen Art und Gattung unterscheiden zu können.

So ist es beispielsweise für die Bildung von monoklonalen Antikörpern von erheblicher Bedeutung, stimulierte Lymphozyten aus einer stimulierte Lymphozyten und nichtstimulierte Lymphozyten enthaltenden Suspension zu bestimmen.

Bestimmte Lymphozyten bilden gegen Fremdstoffe im Organismus Antikörper (Glycoproteine), z.B. gegen ein Fremdeiweiss, das in die Blutbahn injiziert worden ist. Fusioniert man solche zur Bildung und Abgabe von Antikörpern stimulierten Lymphozyten mit einer Tumorzelle, wie der Myelomzelle, so besteht die Chance, dass sich eine sog. Hybridomzelle bildet, die die Eigenschaft beider Elternteile besitzt. Diese Zelle produziert Antikörper, und zwar spezifisch nur gegen den betreffenden Fremdstoff (sog. monoklonale Antikörper). Sie ist praktisch unsterblich und lässt sich im Gegensatz zu einer normalen ausdifferenzierten Zelle, wie dem Lymphozyten, permanent in Nährmedien vermehren.

Da jedoch von der Vielzahl der im Lymphstrom befindlichen Lymphozyten nur eine sehr geringe Anzahl stimuliert werden, aber möglichst nur die stimulierten Lymphozyten der Fusion zugeführt werden sollten, müssen die stimulierten von den nichtstimulierten Lymphozyten in einem Selektionsverfahren getrennt werden. Hierbei macht es jedoch schon Schwierigkeiten, die stimulierten Lymphozyten zu erkennen, da sie sich unter dem Mikroskop von den nichtstimulierten Lymphozyten nicht unterscheiden.

Als weiteres Beispiel sei ausserdem genannt, dass einige Mikroorganismen in der Lage sind, durch Ausscheidung gewisser Zellinhaltsstoffe (hauptsächlich Glycoproteine) lebende Kulturhefen zu lysieren. Diese Toxine werden als «Killerfaktoren» bezeichnet. «Killerhefen» können bei der Bierherstellung Schwierigkeiten verursachen. Bereits bei einem geringen Kontaminationsgrad bewirken sie Änderungen der Vergärung und der Qualität des Bieres.

Die Existenz und Auswirkung von Killerreaktionen wurden sowohl bei Brauereihefen, Weinhefen und Backhefen als auch bei anderen Arten von Zellgattungen, wie z.B. Hansenula, Pichia, Deberyomyces Kluyveromyces, Candida und Torulopsis festgestellt.

In den genannten und vielen anderen Fällen kommt es darauf an, zunächst einmal die sezernierenden Zellen zu erkennen, um weitere Massnahmen einleiten zu können. Diese können darin bestehen, dass, wie beispielsweise im Falle der stimulierten Lymphozyten, diese Zellen anschliessend ausgesondert werden. Im Falle der Killerhefezellen dient das Erkennen solcher Zellen zunächst zur Überwachung des Gärprozesses. Dies wiederum kann dazu führen, dass Massnahmen zur Vermeidung der Kontamination eingeleitet werden.

Es ist Aufgabe der Erfindung, ein Verfahren zu schaffen, das es ermöglicht, Zellen, die Zellinhaltstoffe, wie Proteine und Glycoproteine, Hormone oder Wachstums-Faktoren sezernieren, von Zellen der gleichen Art und Gattung zu unterscheiden.

Diese Aufgabe wird gemäss der Erfindung dadurch gelöst, dass die in einem Medium enthaltenen Zellen elektrischen Drehfeldkräften variabler Drehgeschwindigkeit ausgesetzt werden und die die Zellinhaltstoffe sezernierenden Zellen anhand ihres von den übrigen Zellen unterschiedlichen Rotationsverhaltens bestimmt werden.

Das Verfahren gemäss der Erfindung basiert auf der Entdeckung, dass Zellen, die Zellinhaltstoffe, wie Proteine und/oder Glycoproteine, Hormone oder das Wachstum beeinflussende Stoffe (sogenannte «Wachstums-Faktoren» bzw. «growthfactors») ausscheiden, ein anderes Rotationsverhalten im elektrischen Drehfeld zeigen als nichtsezernierende Zellen der gleichen Art oder Gattung, was auf eine Veränderung der Membran infolge des Durchtritts der Zellinhaltstoffe zurückzuführen ist.

Aus Z. Naturforsch. 37 c, 908–915 (1982), «Rotating-Field-Induced Rotation and Measurement of the Membrane Capacitance of Single Mesophyll Cells of Avena sativa», W.M. Arnold and U. Zimmermann, ist bekannt, dass einzelne Zellen, hier Protoplasten, in einem elektrischen Drehfeld, das beispielsweise von vier jeweils um 90° versetzt angeordneten Elektroden ausgeht, in Rotation gebracht werden können. Es ist ausserdem bekannt, dass einzelne Zellen einer bestimmten Zellart bei einer bestimmten Frequenz des Drehfeldes (der sog. charakteristischen Frequenz) in maximale Rotationsgeschwindigkeit versetzt werden können.

Die charakteristische Frequenz für eine bestimmte Zellart ist zwar von weiteren Parametern abhängig, so von der Intensität des elektrischen Drehfeldes und den Umgebungsbedingungen für die Zellen, der Leitfähigkeit des Mediums, in welchem sich die Zellen befinden, und der Temperatur des Mediums. Für eine Unterscheidungsmessung kommt es jedoch nur auf das unterschiedliche Rotationsverhalten der zu unterscheidenden Zellen, so beispielsweise auf die unterschiedliche charakteristische Frequenz der die Inhaltstoffe ausscheidenden Zellen und der übrigen Zellen der gleichen Art und Gattung an. Die Leitfähigkeit des Mediums, die zweckmässigerweise in einem Bereich von 5 bis $500 \times 10^{-6}$ S $\times$ cm$^{-1}$, insbesondere 5 bis $50 \times 10^{-6}$ S $\times$ cm$^{-1}$ liegt, ist daher für die Unterscheidung der Zellen selbst nur von untergeordneter Bedeutung. Auch die Temperatur des Mediums ist in erster Linie danach einzustellen, dass einerseits die Zellen nicht geschädigt werden, andererseits aber auch eine Bildung und Sekretierung der auszuscheidenden Zellinhaltstoffe möglich ist.

Eine zweckmässige Ausführungsvariante des Verfahrens gemäss der Erfindung besteht darin, dass die sezernierenden Zellen durch Einstellen ihrer höchsten Rotationsgeschwindigkeit bei der in einem vorgegebenen Medium für sie massgeblichen, charakteristischen Frequenz des elektrischen Drehfeldes von den übrigen Zellen unterschieden werden. Dabei werden die in der Suspension befindlichen Zellen unter dem Mikroskop im Hinblick auf ihr Rotationsverhalten beobachtet, wobei beim Verändern des Frequenzbereiches des Drehfeldes zunächst am leichtesten zu beobachten sein wird, bei welcher Frequenz die grosse Zahl der Zellen, nämlich die übrigen Zellen mit ihrer höchsten Geschwindigkeit rotieren. Durch weiteres Verändern der Frequenz des Drehfeldes und Einstellen der charakteristischen Frequenz der sezernierenden Zellen sind diese im allgemeinen jedoch für ein geübtes Auge leicht von den übrigen zu unterscheiden. Dabei ist davon auszugehen, dass der Fachmann im praktischen Fall die charakteristische Frequenz der sezernierenden Zellen und der übrigen Zellen kennen wird, so dass die Unterscheidung der Zellen hierdurch erleichtert wird. Wenngleich – wie bei allen biologischen Populationen – eine natürliche Streubreite der Eigenschaften der Zellen vorhanden ist und diese Streubreite daher auch beim Rotationsverhalten der Zellen festzustellen ist, ist dies für eine Unterscheidung der Teilchen im allgemeinen nicht hinderlich.

Zur Durchführung des Verfahrens gemäss der Erfindung ist eine Vorrichtung geeignet, bei der mindestens drei Elektroden, die, zwischen sich einen Zwischenraum für eine die Zellen enthaltende Kammer oder einen Behälter bildend oder in die zur Aufnahme der Zellen vorgesehene Kammer hineinragend, derart angeordnet sind, dass der Kammer- bzw. Behälterraum einem von den Elektroden ausgehenden elektrischen Drehfeld ausgesetzt ist, sowie durch eine an die Elektroden anschliessbare Einrichtung zur Erzeugung eines Drehfeldes variabler Drehfrequenz. Zweckmässigerweise ist dabei auch die Intensität des Drehfeldes variabel. Sie sollte dabei in einem solchen Bereich liegen, dass in der Kammer etwa 1 bis 1000 V/cm erzielt werden. Der Frequenzbereich sollte im Bereich zwischen 1 Hz und 1 GHz liegen. Bei der Durchführung des Verfahrens gemäss der Erfindung ist eine Intensität zu wählen, die unterhalb der elektrischen Durchbruchspannung für die zu behandelnden Zellen liegt.

Wie sich gezeigt hat, ist es ausserdem zweckmässig, wenn die das Drehfeld erzeugenden elektrischen Spannungen sinusförmig sind. Es ist jedoch auch möglich, das Drehfeld durch rechteckförmige Spannungen oder durch pulsweise abgegebene Spannungen oder Spannungen einer anderen Form zu erzeugen.

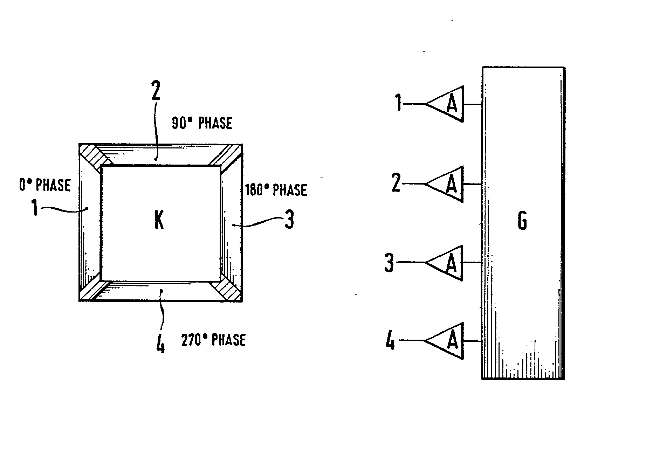

In der Zeichnung ist beispielsweise eine zur Durchführung des Verfahrens gemäss der Erfindung geeignete Vorrichtung schematisch dargestellt und wird im folgenden näher erläutert:

Es sind vier Elektroden vorgesehen, die auf einer in der Zeichnung nicht dargestellten Grundplatte aus elektrisch nicht leitendem Material so angeordnet sind, dass sie die seitlichen Wände der zur Aufnahme der Zellen vorgesehenen Kammer K bilden. Die Elektroden sind an den Ecken der Kammer miteinander und an der Grundplatte mittels eines elektrisch isolierenden Klebemittels verklebt. Die Elektrodenlänge und damit die Länge der Seitenwände der Kammer beträgt 1 mm, die Höhe der Elektroden 0,2 mm. Die quadratische, nach oben offene Kammer K hat somit das Seitenmass von 1 mm und die Höhe von 0,2 mm. Die aus einer Platinfolie bestehenden Elektroden sind in der aus der Zeichnung ersichtlichen Reihenfolge mit den Verstärkern A des Generators G verbunden. Die von den Verstärkern ausgehenden Spannungen sind – wie ebenfalls aus der Zeichnung ersichtlich ist – um jeweils 90° phasenverschoben, wodurch in der Kammer ein elektrisches Drehfeld erzeugt wird.

Allgemeines zu den nachfolgenden Ausführungsbeispielen:

Es wurden stimulierte B-Lymphozyten erzeugt, indem eine Maus durch Schafs-Erythrozyten immunisiert wurde. Die der Maus entnommene Lymphozytenfraktion enthielt die stimulierten Lymphozyten zusammen mit nichtstimulierten Lymphozyten.

Die Lymphozytenfraktion wurde in eine Schicht von Erythrozyten vom Schaf gegeben und später die stimulierten, d.h. den Antikörper sezernierenden Lymphozyten durch Mikropepipettieren wieder entnommen. Die stimulierten Lymphozyten konnten dabei daran erkannt werden, dass sich um sie herum eine Gruppe lysierter Erythrozyten gebildet hatte (nach der in den «Annales of Institute Pasteur, 1975» (Zagury u. Mitarbeiter) beschriebenen Methode).

Auf diese Weise wurden stimulierte Lymphozyten, die einen Antikörper gegen die Erythrozyten vom Schaf sezernierten, gewonnen. Ein Anteil dieser stimulierten Lymphozyten wurde mit mindestens der gleichen Menge nichtstimulierter Lymphozyten, die ebenfalls mittels Mikropipette der Erythrozytenschicht entnommen worden waren, vermischt.

Die Lymphozytenmischung wurde, bevor sie in die Rotationskammer K gegeben wurde, dreimal in einem Medium geringer Leitfähigkeit (0,3 M Mannitol mit Spuren von Natriumchlorid als Elektrolyt) gewaschen.

Ausführungsbeispiel 1

Etwa 10 µl einer das Lymphozytengemisch enthaltenden Suspension wurde in die Rotationskammer K gegeben. Die Leitfähigkeit der Suspension betrug $18,4 \times 10^{-6}$ S/cm$^{-1}$, die Temperatur betrug 35 °C.

Die Suspension wurde einem elektrischen Drehfeld einer Intensität von etwa 100 V/cm ausgesetzt.

Die stimulierten Lymphozyten konnten unter dem Mikroskop an ihrer höheren Rotationsgeschwindigkeit bei einer Drehfrequenz von ungefähr 38±5 kHz erkannt und von den übrigen Lymphozyten unterschieden werden, die bei einer Drehfrequenz von etwa 25±5 kHz auf ihre höchste Rotationsgeschwindigkeit gebracht wurden.

Die aufgrund ihres Rotationsverhaltens als stimulierte Lymphozyten erkannten Lymphozyten wurden durch Mikropipettieren der Rotationskammer entnommen und zur Kontrolle wieder in eine Schicht von Schafs-Erythrozyten gegeben. Die Kontrolluntersuchung bestätigte, dass es sich um stimulierte Lymphozyten handelte.

Ausführungsbeispiel 2

Wie in Ausführungsbeispiel 1 beschrieben, wurde eine Mischung von stimulierten und nichtstimulierten Lymphozyten einem elektrischen Drehfeld ausgesetzt. Die Leitfähigkeit der die Lymphozyten enthaltenden Suspension betrug jedoch $5,2 \times 10^{-6}$ S/cm$^{-1}$.

Auch in diesem Falle wurden die stimulierten Lymphozyten an ihrer maximalen Rotationsgeschwindigkeit erkannt. Die entsprechende Drehfrequenz betrug etwa 12±3 kHz. Die Drehfrequenz, bei der die nichtstimulierten Lymphozyten am schnellsten rotierten, betrug etwa 7±2 kHz.

## Patentansprüche

1. Verfahren zur Unterscheidung von Zellen, die Zellinhaltstoffe, wie Proteine und/oder Glycoproteine, Hormone oder sog. Wachstums-Faktoren sezernieren, von Zellen der gleichen Art oder Gattung, die keine Zellinhaltstoffe sezernieren, dadurch gekennzeichnet, dass die in einem Medium enthaltenen Zellen elektrischen Drehfeldkräften variabler Drehgeschwindigkeit ausgesetzt werden und die die Zellinhaltstoffe sezernierenden Zellen anhand ihres von den übrigen Zellen unterschiedlichen Rotationsverhaltens bestimmt werden.

2. Verfahren nach Anspruch 1, dadurch gekennzeichnet, dass die sezernierenden Zellen durch Einstellen ihrer höchsten Rotationsgeschwindigkeit bei der in einem vorgegebenen Medium für sie massgeblichen, charakteristischen Frequenz des elektrischen Drehfeldes von den übrigen Zellen unterschieden werden.

## Revendications

1. Procédé pour distinguer des cellules qui sécrètrent des substances intracellulaires comme des protéines et/ou des glycoprotéines, des hormones ou ce que l'on appelle des facteurs de croissance, de cellules de même espèce ou même genre qui ne sécrètent pas de substances intracellulaires, caractérisé en ce qu'il consiste à soumettre les cellules contenues dans un milieu à des forces électriques d'un champ tournant de vitesse variable et à déterminer les cellules sécrétant des substances intracellulaires leur comportement à la rotation, qui est différent de celui des autres cellules.

2. Procédé suivant la revendication 1, caractérisé en ce qu'il consiste à distinguer les cellules sécrétant des substances intracellulaires des autres cellules en réglant leur vitesse de rotation la plus élevée pour la fréquence caractéristique du champ électrique tournant qui est prépondérante pour elles dans un milieu donné.

## Claims

1. Method for distinguishing cells which give off cell constituents, such as proteins and/or glycoproteins, hormones, or so-called growth factors, from cells of the same kind or category which do not give off cell constituents, characterised in that the cells, contained in a medium, are subjected to electrical rotating-field forces of variable rotational speed, and the cells giving off cell constituents are detected by means of their rotation behaviour which differs from the other cells.

2. Method according to claim 1, characterised in that the cells which give off cell constituents are distinguished from the other cells by bringing about their maximum rotational speed at the characteristic electrical rotating-field frequency relevant to them in a given medium.

1/·1

**2**

90° PHASE

0° PHASE

**1**

**K**

180° PHASE

**3**

**4**   270° PHASE

**1** ◁A▷

**2** ◁A▷

**3** ◁A▷   **G**

**4** ◁A▷